Europäisches Patentamt

European Patent Office

Office européen des brevets

(19)

(11) Publication number: **0 056 420**

**A1**

(12) # EUROPEAN PATENT APPLICATION

(21) Application number: **81100246.8**

(22) Date of filing: **15.01.81**

(51) Int. Cl.³: **A 61 K 9/06**

(43) Date of publication of application:
**28.07.82 Bulletin 82/30**

(84) Designated Contracting States:
**AT BE CH DE FR GB IT LI LU NL SE**

(71) Applicant: SCHERING CORPORATION
2000 Galloping Hill Road
Kenilworth, New Jersey 07033(US)

(72) Inventor: Chrai, Sukhbir Singh
10 Honiss Street
Belleville New Jersey 07109(US)

(72) Inventor: Gupta, Santosh Kumar
11 George Street
Bloomfield New Jersey 07003(US)

(72) Inventor: Hensley, Michael Jack
6272 Sunnyspring
Columbia Maryland 21044(US)

(74) Representative: Antony, Fritz, Dr. et al,
P.O. Box 601 Winkelriedstrasse 35
CH-6002 Lucerne(CH)

(54) **Ophthalmic gel.**

(57) A topical ophthalmic gel comprising at least one opthalmic medicament, polyvinyl alcohol, a borate gelling agent, and sterile water; said gel being maintained at a pH of 6.5-8.5, preferably 6.9-8.5. This ophthalmic gel is a long-acting, topical medicament which has a pH that is compatible with even injured eyes and has uniform release characteristics over a long period of time.

EP 0 056 420 A1

OPHTHALMIC GEL

Most ophthalmic medicaments are topically administered to the eye. The most common dosage form for such medicaments is liquid drops. The liquid drop form is easy to apply, but suffers from the inherent disadvantage that the drug it contains is rapidly washed from the precorneal ocular cavity by tear flow. Thus, a continued sustained drug level is not obtained. Sustained levels are typically attained by periodic application of the drops, but this results in frequent administrations by the patient. The result of frequent administration and washing by tear flow is that the level of medication surges to a peak at the time the drops are applied, then the drug concentration falls rapidly.

Other methods of applying ophthalmic medicaments are the unitary ocular inserts. While such inserts deliver the drug in a sustained manner, they suffer from the disadvantages of being difficult to insert and remove, and are expensive.

The prior art discloses a polyvinyl alcohol-boric acid-iodine complex useful in the treatment of eye infections. [Klin.Oczna., 36 (1), 27-32 (1966), C.A.67:72404r.]

However, the researchers concluded that the complex must be maintained at a pH of 5.0-5.5 to afford stability of the iodine. At this pH, the complex is a conventional solution dosage form. Also, this pH is not compatible for use with injured eyes.

A high viscosity gel containing pilocarpine has been described as being useful in topical treatment of increased intraocular pressure (American Journal of Ophthalmology 88, 843-846, 1979), however, no details about the pilocarpine gel are disclosed.

The present invention relates to a topical ophthalmic gel for use in the eyes of human and domestic animals comprising 0.05-10% (preferably 0.05-2.%) by weight of at least one ophthalmic medicament, 1-3% by weight of a polyvinyl alcohol of the grades 20 to 100, with a molecular weight of over 10,000 and a viscosity rating of 5 to 65 centipoises (measured in 4% aqueous solution at 20°C), 0.1-1% by weight of a borate gelling agent; and 85-99% sterile water; said gel being maintained at a pH of 6.5-8.5, preferably 6.9-8.5.

This ophthalmic gel is a long-acting, topical medicament which has a pH that is compatible with even injured eyes.

Because the polyvinyl alcohol gel is formed with the medicament already in solution the medicament is uniformly dispersed and consequently the medicament has more uniform release characteristics over a longer period of time than ophthalmic medicaments known in the art.

Thus the gel of this invention has an improved biological response and an increased duration of activity. This medicament also provides a dosage form which is more convenient and less messy to use than conventional eye drops. Additionally, the gel is selfdissolving, that is, the tear flow gradually dissolves the gel without leaving a residue. As is described further below in detail the shelf life of the gel of this invention is at least 1 to 3 years. If the gel contains an active ingredient which is not sufficiently stable at the pH of 6.5 to 8.5 the components of the composition are kept in appropriate combination in two separate containers and the desired medicament is prepared by mixing these components shortly before the treatment is started.

Ophthalmic drugs suitable for incorporation into the gel of the present invention include, but are not limited to, antibiotics such as tetracycline, chlortetracycline, bacitracin, neomycin, polymixin, gramicidin, oxytetracycline, chloramphenicol, gentamicin, sisomicin, penicillin, netilmicin, rosaramicin and erythromycin; antibacterials such as sulfonamides, sulfacetamide, sulfamethizole —————————— and sulfisoxazole; antivirals such as idoxuridine and vidarabine; other antibacterial agents such as nitrofurazone and sodium propionate; antiallergenics such as antazoline, methapyriline, chlorpheniramine, pyrilamine and prophenpyridamine; anti-inflammatories such as hydrocortisone, hydrocortisone acetate, dexamethasone, dexamethasone 21-phosphate, fluocinoline, medrysone, prednisolone, methyl-prednisolone, prednisolone 21-phosphate, prednisolone acetate, fluorometholone, betamethasone, betamethasone valerate, triamcinolone, indomethacin and flunixin; decongestants such as phenylephrine, naphazoline, and tetrahydrozoline; miotics and anticholinesterases such as pilocarpine, eserine salicylate, carbachol, di-isopropyl fluorophosphate, phospholine iodide, and demecarium bromide; mydriatics such as atropine sulfate, cyclopentolate, homatropine, scopolamine, tropicamide, eucatropine and hydroxyamphetamine; and sympathomimetics such as epinephrine.

The ophthalmic drug may be in formulation in its base form, or, optionally in a salt form. Where a salt form is utilized the salt may be any eye-compatible, pharmaceutically acceptable acid addition salt. Such salts can be derived from a variety of organic and inorganic acids such as sulfuric, phosphoric, hydrochloric, hydrobromic, hydriodic, sulfamic, citric, lactic, maleic, malic, succinic, tartaric, cinnamic, acetic, benzoic, gluconic, ascorbic, and nitric. Preferred salts are those utilized in the U.S. Pharmacopeia, XIX edition, given for each individual drug. For instance, atropine is conventionally and preferably formulated as the sulfate, homatropine as the hydrobromide, pilocarpine as the hydrochloride or the nitrate, epinephrine as the bitartrate, physostigmine as the salicylate or sulfate, flunixin as the N-methyl glucamine, gentamicin as the sulfate, and so on. For example, a preferred gel contains a combination of betamethasone phosphate and gentamicin sulfate.

The polyvinyl alcohol suitable for use in the present invention is typically of the grades of 20 to 100, with a molecular weight of over 10,000 and a viscosity rating of 5 to 65 centipoises (measured in 4% aqueous solution at 20$^{o}$C). A preferred polyvinyl alcohol is Gelvatol$^{R}$ 20-90 (Monsanto) having a viscosity of 35 to 45 centipoises (measured in 4% aqueous solution at 20$^{o}$C), and a molecular weight of approximately 125,000.

The gelling agent utilized in this invention to effect formation of the gel may be any of the borate salts such as sodium borate, potassium borate, calcium borate or magnesium borate. Preferably, sodium or potassium borate is used.

Alternatively, the borate may be generated in situ by the reaction of boric acid with aqueous alkali. This is particularly advantageous in the instance where it is desirable to package the components of the gel, i.e., polyvinyl alcohol solution and the borate solution separately. The boric acid may be present in the polyvinyl alcohol solution without effecting formation of the gel. Upon addition of a second solution containing aqueous alkali, the gelling occurs. Suitable aqueous alkali solutions for use in combination with the boric acid include, but are not limited to, sodium hydroxide, potassium hydroxide, sodium carbonate and potassium carbonate. Sodium hydroxide is preferred for use in the invention.

Suitable buffering agents for use in the present invention are those which are typically used in ophthalmic medicinals. Included, but not limited are buffering combinations such as boric acid-sodium acetate and preferably boric acid-sodium borate. Other salts useful in buffer combinations include sodium and potassium bicarbonate, potassium carbonate and sodium citrate. Most preferably, the amounts of the buffer's components are adjusted so as to afford a final pH of about 7.4.

Specifically the amount of buffer can range from about 0.1 to 5%, preferably 0.1 to 1%, wherein percentages are by weight based upon the total weight of the overall composition. The ratio of the components is balanced to provide the desired pH for the overall composition.

The gel is usually adjusted in a conventional manner so as to provide an isotonic gel, i.e., a gel wherein the sodium chloride equivalent is about 0.9% by weight of the gel. Preferably, sodium chloride is utilized to adjust the percentage of salt components.

The medicated gel of the present invention may be optionally preserved by the addition of conventional levels of ophthalmic preservatives such as benzalkonium chloride (0.013%), benzethonium chloride (0.01%), phenylmercuric acetate (0.004%), phenylmercuric nitrate (0.004%), chlorobutanol (0.5%), β-phenylethyl alcohol (0.5%) or thiomersal, (0.01%).

The ophthalmic medicament is present in the medicated gel in amounts ranging from about 0.05-10% by weight. Particularly suitable amounts for incorporation for the individual drug may be determined by reference to the U.S. Pharmacopeia, XIX edition (1970), Remington's Pharmaceutical Sciences, 14th edition (1970), or Goodman and Gilman, The Pharmacological Basis of Therapeutics, 5th ed., (1976).

Typical preferred gels contain the ophthalmic medicament in the amounts given in Table I below.

### Table I

| Drug | Preferred Concentration Range (in %) |
|---|---|
| Antazoline Phosphate | 0.25-0.5 |
| Atropine Sulfate | 0.5 -4.0 |
| Benoxinate Hydrochloride | 0.2 -0.4 |

Table I (continued)

| | |
|---|---|
| Betamethasone Phosphate | 0.05-0.5 |
| Carbachol | 0.75-3.0 |
| Cocaine Hydrochloride | 2.0 -4.0 |
| Cyclopentolate Hydrochloride | 0.5 -2.0 |
| Epinephrine Bitartrate | 1.0 -2.0 |
| Fluorescein Sodium | 1.0 -2.0 |
| Flunixin NMG (N-methyl glucamine) | 0.5 -2.0 |
| Gentamicin Sulfate | 0.1 -0.5 |
| Homatropine Hydrobromide | 2.0 -5.0 |
| Hydroxyamphetamine Hydrobromide | 0.2 -1.0 |
| Idoxuridine | 0.05-0.1 |
| Lidocaine Hydrochloride | 1.0 -4.0 |
| Neostigmine Bromide | 1.2 -2.5 |
| Phenylephrine Hydrochloride | 0.1 -2.5 |
| Physostigmine Salicylate | 0.25-0.5 |
| Physostigmine Sulfate | 0.25-0.5 |
| Pilocarpine Hydrochloride | 0.25-5.0 |
| Pilocarpine Nitrate | 0.5 -3.0 |
| Scopolamine Hydrobromide | 0.2 -0.5 |
| Tetracaine Hydrochloride | 0.25-0.5 |
| Vidarabine | 1.0 -3.0 |

Optionally, the gel of the present invention may also
contain up to 1% of a conventional eye-compatible stabilizing agent. Suitable stabilizing agents for use in the present invention include, but are not limited to, EDTA, sodium bisulfite, sodium metabisulfite, and thiourea. Most preferably, the stabilizing agent is present in an amount of 0.1-0.2% by weight of the gel.

The gel of the present invention has a shelf life which is dependent upon the particular ophthalmic drug incorporated therein. In the case of drugs which are normally stable in solution of pH 7-9, the usual shelf life of the gel of this invention is at least 1-3 years. For instance, gentamicin sulfate may be formulated into a gel with a shelf life of at least 2 years,

For drugs which are most stable at lower pH's e.g., 4-7, the shelf life of the gel after formulation is about 3-12 months. For instance, pilocarpine is most stable at a pH of about 4.5 to 5.5. The pH is not suitable for use in the eye since to minimize irritation the pH should preferably be about 6 to 8 and most preferably about 7.4. As noted by Remington's Pharmaceutical Sciences, 14th edition, p. 1553, pilocarpine hydrochloride solutions have been shown to need a minimum pH of 6.8 for reasonable eye comfort. However, at a pH of 6.8, the time for 50% decomposition of pilocarpine at 25°C is 66 days. At elevated temperatures (120°C) 50% decomposition of pilocarpine in a solution of this pH occurs in about 34 minutes. At a pH of 6.8, pilocarpine is obviously not stable enough for normal shelf life or for autoclaving. Thus, the use of pilocarpine ophthalmic solutions at a pH of 6.8 or above is usually limited to situations where it is possible to constantly prepare fresh solutions of the eye drops for use by the patient by sterile filtration, and have an expiration date of 1 month to take care of the shelf-life problem. Alternatively, a solution having a pH of about 5 is utilized with a long expiration date but which may not be used in cases where the eye has been injured.

To obviate such problems, a gel containing pilocarpine or a salt thereof would be prepared by the dispensing pharmacist just prior to purchase by the patient. Thus, as a preferred embodiment of our invention, the gel is prepared by mixing a sterile solution of the pilocarpine or salt thereof and the polyvinyl alcohol with a sterile, aqueous solution of the borate gelling agent. The pilocarpine/polyvinyl alcohol solution is maintained at a pH of about 4-5 to maintain the stability of the pilocarpine. This pH may be obtained by dissolving the pilocarpine salt in water or, if necessary due to the presence of other excipients, by the addition of a suitable buffer. The borate gelling agent solution contains sufficient buffers so that when the two solutions are mixed to form the gel, the resultant .pH will be 6.5-8.5. Depending on the nature of the pilocarpine/polyvinyl alcohol solution, the borate gelling agent solution may or may not require additive buffers to attain this pH. Other medicaments can be stored and prepared in an analogous way.

It is useful to supply these medicaments in a kit of parts, which preferably comprises two containers. These house the borate gelling agents solution and the drug-polyvinyl alcohol solution, respectively. These units are delivered from the manufacturer in a sterile condition. It is useful to also provide a dispensing-tip and a sterile closure therefor in the kit.

A preferred kit of parts for producing a gel containing an acid stable drug will thus comprise a first container ("container B") housing an aqueous solution containing the ophthalmic medicament and a polyvinyl alcohol, said first solution maintained at a pH to afford stability of the ophthalmic medicament, and a second container ("container A") housing an aqueous solution containing a gelling agent. The first solution contains the ophthalmic medicament in an amount sufficient to make 0.05-10% by weight when the first solution is mixed with the second solution and the polyvinyl alcohol in an amount sufficient to make 1.3% by weight when the first solution is mixed with the second solution. The second solution contains the borate gelling agent in an amount sufficient to make 0.1-1% by weight when the first solution is mixed with the second solution. The second solution additionally contains buffers in an amount sufficient to afford a pH of 6.5-8.5 when the first solution is mixed with the second solution. If a boric acid and alkali combination is utilized to provide the borate gelling agent, the boric acid is placed in "container B" and the alkali in "container A". Preferably, the two solutions are packaged in a sterile condition. Prior to dispensing to the patient, the pharmacist, doctor or nurse, prepares the gel by merely adding the contents of "container A" to "container B".

A dispensing tip may then be placed on the "container B" and covered by a sterile closure prior to dispensing to the patient. The patient thus receives a container of the gel which is stable for at least 3 months and which is in an eye-comfortable and convenient dosage form. Most advantageously, the dispensing pharmacist is provided with an eye-compatible system which is in a sterile form when he receives it and thus requires no further sterilization prior to dispensing to the patient.

The gel container will typically be of a suitable size so as to provide an amount of gel sufficient for a multitude of drug applications. The patient is thus provided with a prescription which does not have to be frequently refilled.

The gel itself is well-suited to patient self-administration since its viscous nature provides a product which does not spill, drip or flow unwantedly from its dispenser. Additionally, and most advantageously, the gel form provides an improved therapeutic effect of enhanced duration. Thus, a patient using a pilocarpine gel will not experience the blurriness of vision towards the end of a treatment period such as is normally experienced by patients using pilocarpine eye drops. This blurriness occurs when the effects of the pilocarpine begin to wear off. Such a side effect is a severe handicap to glaucoma patients using pilocarpine eye drops on a regular and long-term basis since it prevents or restricts normal activities such as reading, sewing and driving. Also advantageously, the enhanced therapeutic effect of the gel dosage form enables the dose of pilocarpine to be lowered, typically to about a 1% pilocarpine gel, while retaining the same degree of biological response.

A gel containing 1.0% flunixin NMG likewise provides a suitable anti-inflammatory response while a gel containing 0.2% gentamicin sulfate provides sufficient antibacterial effects.

When preservatives, stabilizers or agents for adjusting the tonicity are used in the gel, and the gel is packaged in two separate containers, such preservatives or stabilizers may be present in either or both of the solutions. Preferably, however, they are contained in the solution containing the polyvinyl alcohol.

The required daily dosage of the ophthalmic gel will depend on the patient's individual condition and the

- 16 -

particular ophthalmic ailment disease state for which the gel is being employed. Typically, 0.05 to 0.8 ml of the gel will be administered 1-3 times per day.

The following examples describe in detail compositions illustrative of the present invention and methods which have been devised for their preparation. It will be apparent to those skilled in the art that many modificiations, both of materials and methods, may be practiced without departing from the purpose and intent of this disclosure.

EXAMPLE 1

A pilocarpine gel is prepared by mixing 1 part by volume of a solution consisting of:

| | | |
|---|---|---|
| Pilocarpine hydrochloride | 10.0 | mg |
| Polyvinyl alcoho, Grade 50-42 available from Dupont | 40.0 | mg |
| Water, sterile USP q.s. | 1.0 | ml |

with 1 part by volume of a solution consisting of:

| | | |
|---|---|---|
| Sodium borate | 7.6 | mg |
| Water, sterile USP q.s. | 1.0 | ml |

The final gel has a concentration of 0.5% pilocarpine hydrochloride and a pH of 6.9 - 8.5.


EXAMPLE 2

Substitution of 20.0 mg of pilocarpine hydrochloride for the 10.0 mg of pilocarpine hydrochloride of Example 1 and repetition of the procedure detailed therein affords a medicated gel having a pilocarpine concentration of 1.0% and a pH of 6.9 - 8.5.


EXAMPLE 3

A pilocarpine hydrochloride gel is prepared by mixing 1 part by volume of a solution consisting of:

| | | |
|---|---|---|
| Pilocarpine hydrochloride | 5.0 | mg |
| Polyvinyl alcohol, Grade 99-55 available from Baker | 40.0 | mg |

| Water, sterile USP | q.s. | 1.0 | ml |

with 1 part by volume of a solution consisting of:

| Sodium borate | | 7.6 | mg |
| Water, sterile USP | q.s. | 1.0 | ml |

The final gel has a concentration of 0.25% pilocarpine hydrochloride and a pH of 6.9 - 8.5.

EXAMPLE 4

A pilocarpine nitrate gel is prepared by mixing 1 part by volume of a solution consisting of:

| Pilocarpine nitrate | | 10.0 | mg |
| Polyvinyl alcohol, Grade 89-6 available from Baker | | 30.0 | mg |
| Water, sterile USP | q.s. | 1.0 | ml |

with 1 part by volume of a solution of:

| Sodium borate | | 7.6 | mg |
| Water, sterile USP | q.s. | 1.0 | ml |

The pilocarpine nitrate concentration of the resulting gel is 0.5% and the pH is 6.9 - 8.5.

EXAMPLE 5

A pilocarpine hydrochloride gel is prepared by mixing 1 part by volume of a solution consisting of:

| Pilocarpine hydrochloride | | 10.0 | mg |
| Polyvinyl alcohol, Grade 50-42 available from Dupont | | 40.0 | mg |

Boric acid                                              5.0  mg

Water, sterile USP                          q.s.        1.0  ml with 1 part by volume of a solution of

Sodium hydroxide                                        0.4  mg

Water, sterile USP                          q.s.        1.0  ml

The pilocarpine hydrochloride concentration of the resulting gel is 0.5% and the pH is 6.9 - 8.5.


EXAMPLE 6

A pilocarpine hydrochloride gel is prepared by mixing 1 part by volume of a solution consisting of:

Pilocarpine hydrochloride                              10.0  mg

Polyvinyl alcohol, Grade 50-42                         40.0  mg
available from Dupont

Boric acid                                   .          2.0  mg

Water, sterile USP                          q.s.        1.0  ml with 1 part by volume of a solution of:

Sodium borate                                           4.0  mg

Water, sterile USP                          q.s.        1.0  ml

This gel has a pilocarpine hydrochloride concentration of 0.5% and a pH of 6.9 - 8.5.


EXAMPLE 7

A gel is prepared by mixing 1 part by volume of a solution consisting of:

Gentamicin sulfate                                      4.0  mg

Polyvinyl alcohol, Grade 50-42                    40.0  mg
available from Dupont

Water, sterile USP                      q.s.      1.0  ml with 1 part by volume of a solution consisting of

Sodium borate                                     7.6  mg

Water, sterile USP                                1.0  ml

The final gel has a concentration of 0.2% gentamicin sulfate and a pH of 6.9 - 8.5.


EXAMPLE 8

A gel is prepared by mixing 1 part by volume of a solution consisting of:

Flunixin NMG (N-methyl glucamine)                 2.0  mg

Polyvinyl alcohol, Grade 50-42                   40.0  mg
available from Dupont

Water, sterile USP                      q.s.      1.0  ml with 1 part by volume of a solution consisting of:

Sodium borate                                     7.6  mg

Water, sterile USP                      q.s.      1.0  ml

The final gel has a concentration of 1.0% flunixin and a pH of 6.9 - 8.5.


EXAMPLE 9

A gel is prepared by mixing 1 part by volume of a solution consisting of:

Betamethasone phosphate                           2.0  mg

Polyvinyl alcohol, Grade 50-42                   40.0  mg
available from Dupont

| Water, sterile USP | q.s. | 1.0 | ml |

with 1 part by volume of a solution consisting of:

| Sodium borate | | 7.6 | mg |
| Water, sterile USP | q.s. | 1.0 | ml |

The final gel has a concentration of 0.1% betamethasone phosphate and a pH of 6.9 - 8.5.

EXAMPLE 10

A pilocarpine hydrochloride gel is prepared by mixing 2 parts by volume of a solution consisting of:

| Pilocarpine hydrochloride | | 13.0 | mg |
| Polyvinyl alcohol, Gelvatol$^R$ 20-90 available from Monsanto | | 25.0 | mg |
| Water, sterile USP | q.s. | 1.0 | ml |

with 1 part by volume of a solution of:

| Sodium borate (10 $H_2O$) | | 20 | mg/ml |

The pilocarpine hydrochloride concentration of the resulting gel is 0.86% and the pH is 7.4.

EXAMPLE 11

A gel is prepared by mixing 10 parts by volume of a solution consisting of:

| Betamethasone sodium phosphate | | 2.0 | mg |
| Polyvinyl alcohol, Gelvatol$^R$ 20-90 available from Monsanto | | 25.0 | mg |
| Boric Acid | | 3.0 | mg |

Water, sterile USP                           q.s.      1.0   ml with 1.2 parts by volume of a solution consisting of:

Sodium borate (10 $H_2O$) solution                     9.0 mg/ml

Water, sterile USP                                     1.0   ml

The final gel has a concentration of 0.2% betametha-sone phosphate and a pH of 7.3.


EXAMPLE 12

A gel is prepared by mixing 10 parts by volume of a solution consisting of:

Gentamicin sulfate                                    10.0   mg

Polyvinyl alcohol,Gelvatol[R] 20-90                   25.0   mg
  available from Monsanto

Boric Acid                                             2.0

Water, sterile USP                           q.s.      1.0   ml with 2 parts by volume of a solution consisting of:

Sodium Hydroxide                                       5.0   mg

Water, sterile USP                                     1.0   ml

The final gel has a concentration of 0.86% gentamicin sulfate and a pH of 7.4.

WHAT IS CLAIMED IS:

1.     A topical ophthalmic gel comprising:

0.05 to 10% (preferably 0.05 to 2%) by weight of at least one ophthalmic medicament; 1-3% by weight of a polyvinyl alcohol of the grades 20-100, with a molecular weight of over 10,000, and a viscosity rating of 5-65 centipoises (measured in 4% aqueous solution at $20^{o}C$); 0.1-1% by weight of a borate gelling agent; and 85-99% sterile water; said gel being maintained at a pH of 6.5 to 8.5, preferably 6.9 to 8.5.

2.     A gel according to claim 1 wherein the borate gelling agent is an alkali metal or alkaline-earth borate salt or is generated in situ by the reaction of boric acid with aqueous alkali.

3.     A gel according to claim 1 or 2 wherein the ophthalmic medicament is pilocarpine hydrochloride, pilocarpine nitrate, gentamicin sulfate, flunixin N-methyl glucamine, betamethasone phosphate, dexamethasone phosphate, or a combination of betamethasone phosphate and gentamicin sulfate.

4.     A gel according to any one of claims 1 to 3 wherein the borate gelling agent is sodium borate.

5.      A gel according to any one of claims 1 to 3 wherein the borate gelling agent is generated in situ by boric acid and sodium hydroxide.

6.      A gel according to any one of claims 1 to 5 wherein the ophthalmic medicament is present in an amount of 0.2-0.5 percent by weight.

7.      A gel according to any one of claims 1 to 6, wherein the pH is 7.0-8.5, preferable 7.4.

8.      A gel according to claim 1, 2, 4, 5 or 6, wherein the ophthalmic medicament is gentamicin sulfate and the pH is 6.9 to 8.5.

9.      A kit of parts for producing a gel of any one of claims 1 to 8 comprising:
- a first container housing an aqueous solution comprising the ophthalmic medicament and the polyvinyl alcohol said first solution maintained at a pH to afford stability of the ophthalmic medicament; and

- a second container housing an aqueous solution comprising the gelling agent;

wherein said first solution contains the ophthalmic medicament in an amount sufficient to make 0.05-10% by weight when said first solution is mixed with said second solution;

wherein said first solution contains the polyvinyl alcohol in an amount sufficient to make 1-3% by weight when said first solution is mixed with said second solution;

wherein said second solution contains the borate gelling agent in an amount sufficient to make 0.1-1% by weight when said first solution is mixed with said second solution; and

wherein said second solution contains buffers in an amount sufficient to afford a pH of 6.5-8.5 when said first solution is mixed with said second solution.

0056420

European Patent
Office

**EUROPEAN SEARCH REPORT**

Application number

EP 81 10 0246

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | US - A - 4 131 651 (SHA.B)<br>* Column 1, line 23 - column 2, line 26; column 2, line 40 - line 48; column 3, line 49 - column 4, line 63 *<br><br>-- | 1,2,3-7 | A 61 K 9/06 |
| | GB - A - 2 042 338 (OTSUKA)<br>* Page 4, lines 18-64 *<br><br>-- | 1,2,4-7 | |
| | US - A - 4 230 690 (JAMAUCHI)<br>* Column 1, lines 6-10; column 1, lines 42-57; column 2, line 50 - column 3, line 34; example 2; claims 1-5 *<br><br>-- | 1,3,6 | TECHNICAL FIELDS SEARCHED (Int. Cl.³)<br><br>A 61 K 9/06 |
| | CHEMICAL ABSTRACTS, vol. 76, no. 26, 26-06-1972, page 326, abstract 158294c<br>Columbus, Ohio, US<br>B. BEDO et al.: "Use of poly(vinyl alcohol) in pharmaceutical practice. I. Poly(vinyl alcohol) used in the preparation of ophthalmic solutions"<br>& Rev. Med. (Tirgu-Mures) 1971, 17(3-4), 448-52<br>* Abstract *<br><br>-- | 1,3 | |
| A | CHEMICAL ABSTRACTS, vol. 85, no. 2, 12-07-1976, page 320, abstract 10336h<br>Columbus, Ohio, US | 3 | CATEGORY OF CITED DOCUMENTS<br><br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | ./. | | &: member of the same patent family, corresponding document |

The present search report has been drawn up for all claims

| Place of search | Date of completion of the search | Examiner |
|---|---|---|
| The Hague | 14-09-1981 | CONTET |

EPO Form 1503.1   06.78

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | "Gentamicin ophthalmic solution" & Fed. Regist. 02-04-1976, 41(65), 14188-9 <br> * Abstract * <br><br> ---- | | |
| | | | TECHNICAL FIELDS SEARCHED (Int. Cl ³) |